# EUROPEAN PATENT SPECIFICATION

(11) **EP 1 558 353 B1**
(45) Date of publication and mention of the grant of the patent: **15.06.2016**
(21) Application number: 03778140.8
(22) Date of filing: 05.11.2003
(51) Int. Cl.: C07C 303/06, C07C 309/04, C07C 29/00, C07C 31/04, C01B 3/34

(54) **ANHYDROUS CONVERSION OF METHANE AND OTHER LIGHT ALKANES INTO METHANOL AND OTHER DERIVATIVES, USING RADICAL PATHWAYS AND CHAIN REACTIONS WITH MINIMAL WASTE PRODUCTS**
WASSERFREIE UMWANDLUNG VON METHAN UND ANDEREN LEICHTEN ALKANENIN METHANOL UND ANDERE DERIVATE UNTER VERWENDUNG VON RADIKALWEGEN UNDKETTENREAKTIONEN MIT MINIMALEN ABFALLPRODUKTEN
CONVERSION ANHYDRE DE METHANE ET D'AUTRES ALCANES LEGERS EN METHANOL ET D'AUTRES DERIVES AU MOYEN DE TRAJETS DE RADICAUX ET DE REACTIONS EN CHAINE PRODUISANT UN MINIMUM DE DECHETS

(30) Priority: 05.11.2002 US 424091 P; 21.06.2003 US 480183 P
(43) Date of publication of application: 03.08.2005
(73) Proprietor: Richards, Alan K., Palm City, Florida 34990 (US)
(72) Inventor: Richards, Alan K., Palm City, Florida 34990 (US)
(74) Representative: Ettmayr, Andreas
(86) International application number: PCT/US2003/035396
(87) International publication number: WO 2004/041399

(56) References cited:
- WO-A-2005/069751
- GB-A- 632 820
- US-A- 2 492 983
- US-A- 2 493 038
- US-A- 2 553 576
- JIN WOO CHUN ET AL: 'Free-radical kinetic model for homogeneous oxidation of methane to methanol' INDUSTRIAL & ENGINEERING CHEMISTRY RESEARCH vol. 32, no. 5, 01 May 1993, pages 796 - 799, XP055029120 DOI: 10.1021/ie00017a005 ISSN: 0888-5885

## Description

### BACKGROUND

This invention relates to organic chemistry, and is relevant in the context of converting methane (or other light alkanes) into methanol or other derivatives. Despite intensive efforts by numerous research teams over at least 60 years, no one previously succeeded in discovering and identifying an efficient and economical chemical reaction scheme for converting methane into methanol or other derivatives.

Methane (CH₄) is a volatile and explosive gas, and its presence in crude oil renders the crude oil more difficult, dangerous, and expensive to handle and transport. If crude oil is to be pumped into a pipeline, a high methane content would lead to the formation of large gas bubbles in the pipeline. These gas bubbles would begin acting in a manner comparable to springs, and each stroke of a pump would simply compress the bubbles, rather than driving the liquid forward. Therefore, methane must be removed before crude oil can be pumped into a pipeline.

Methane also must be removed before crude oil can be loaded into a tanker or holding tank. Otherwise, the high pressures generated by the methane gas, as it tries to escape from the oil, would require stronger and more expensive tanks and expensive vapor-handling systems, and the constant emission of methane from the oil would greatly increase the risk of explosion or fire.

In many oil fields around the world (including many offshore oil fields and platforms), there are no gas pipelines nearby that can transport methane gas to commercial markets. As a result, methane produced at those oil fields is usually referred to by terms such as stranded, waste, remote, or distressed gas. Relatively small portions of it are burned as fuel, to keep the oil-producing equipment running, but large volumes of gas remain left over and cannot be efficiently utilized. The gas cannot be released into the atmosphere, because it can create an explosion risk near the release site, and because it is roughly 20 times more potent as carbon dioxide, on a per weight basis, in contributing to the greenhouse effect and global warming.

Therefore, every month, billions of dollars worth of methane gas must be disposed of, as waste gas. Much of it is burned in flares that emit large quantities of carbon dioxide, a greenhouse gas that aggravates the problem of global warming. Large quantities are also injected back into the ground.

Another major source of "stranded" gas is located in natural gas fields that do not produce large quantities of crude oil. Numerous such gas fields are known to exist in various locations around the world, but the gas cannot be extracted from them and then transported to distant commercial markets in an economically feasible manner.

Still other sources of unused methane are located at coal mines, since methane is commonly associated with coal. In addition, smaller quantities of methane are produced at various types of waste-handling facilities, including landfills that handle organic wastes, and livestock facilities that handle large quantities of animal manure. Although such sources are frequently excluded when "stranded" gas is being discussed by those who are focusing on the huge reserves of stranded gas at remote oil and gas fields, the numerous smaller sources nevertheless add up to very large quantities on a global level.

Many people and companies have attempted to create various methods for using "stranded" methane gas; however, all such efforts have been severely limited by very high expenses and operating costs. As one example, specially-designed tanker ships have been built to transport "liquified natural gas" (LNG) across oceans. Liquified natural gas requires extremely cold ("cryogenic") temperatures, far below the freezing point of water (LNG typically is transported at temperatures of roughly -260° Fahrenheit). Therefore, LNG tanker ships typically contain three or four huge spherical insulated tanks (often referred to as "thermos bottles"), which typically are partially visible as a row of three or four giant semi-circles above the decks of these types of ocean-going ships.

Specialized LNG tankers, the manufacturing facilities required to refrigerate natural gas down to hundreds of degrees below zero, and the specialized types of pumps and pipelines that are required to store, handle, and pump LNG in a safe manner, are extremely expensive, costing multiple billions of dollars for a large facility and a fleet of tankers. Just as importantly, they burn up a large fraction of the feedstock methane, to drive the cooling and heating equipment. To drive the refrigeration systems that will chill methane to a temperature where it will liquify, such facilities uusally burn about 40% of their total methane feedstock; then, once the liquified methane has been loaded onto a tanker, shipped to a destination port, and unloaded from the tanker, another substantial portion of the methane must be burned, in order to re-vaporize the remainder and bring it up to reasonable operating temperatures that can be handled by conventional pipelines. Therefore, LNG systems typically must burn up almost half of their entire starting feedstock, in order to deliver the other half to a commercial market.

Similar operating economics also apply to a product that is usually called synthesis gas, synthetic gas, or simply "Syngas". This gas is mainly a mixture of carbon monoxide, and hydrogen. It can be created by using methane as the major feedstock, usually by "steam reforming" using a nickel catalyst, but this process requires large amounts of energy to drive it. Therefore, roughly 20 to 30% of a natural gas supply must be burned, to convert the remainder into Syngas. The waste problem is aggravated even more, because the resulting Syngas typically requires another highly endothermic (energy-consuming) reaction to convert the Syngas into liquid hydrocarbons and/or alkane hydroxides (e.g., methanol, etc.). Syngas processing is described in numerous articles, patents, and chapters of books (see, e.g., Olah, Hydrocarbon Chemistry, 1995, at p. 15).

Other efforts to create economic uses for "stranded" methane involve on-site conversion of the methane into useful chemical feedstocks. As one example, the hydrogen atoms from methane molecules can be chemically processed to convert nitrogen (present as N₂ in the atmosphere) into ammonia (NH₄). The ammonia can then be converted into fertilizer. However, this is not an efficient use, and it has not become widespread, even in developing countries that badly need fertilizers.

Since none of the above reactions are efficient, many researchers have tried to find ways to convert methane into methanol (i.e., methyl alcohol). Methanol has a formula that can be written as CH₃OH, as H₃COH, or H₃C-OH; as indicated by all three formulas, it has three hydrogen atoms, and a hydroxy group (-OH), all bonded to a central carbon atom. The preferred formula herein is H₃C-OH, since that version helps focus attention on a certain bond.

For similar reasons, various formulas herein are written in ways that focus attention on certain components or bonds. For example, the preferred formula herein for methanesulfonic acid is H₃C-SO₃H, and the preferred formula herein for Marshall's acid is HO₃SO-OSO₃H, rather than the simpler version H₂S₂O₈.

In principle, converting methane into methanol is a highly appealing reaction, for two reasons. First, it merely requires adding a single oxygen atom to methane, and oxygen atoms are abundantly available from the atmosphere. Second, methanol is a liquid that can be easily and safely handled, at normal temperatures and pressures that do not require special and expensive equipment to reach cryogenic temperatures or sustain very high pressures.

The methanol pathway is even more appealing, since methanol has numerous important and valuable uses. As one example, it is a good, clean-burning fuel in its own right, and can be used directly in internal combustion engines that have been "tuned" for methanol; indeed, it is the fuel of choice for certain types of race cars, including high-powered dragsters that need a clean-burning fuel. Since the engines in those cars usually are run for only very short periods of time, they need a clean-burning fuel that will not generate particulates or residues that will gradually accumulate and foul an engine. Therefore, if a sufficient supply of methanol were available, it could be burned as the primary or sole fuel, in clean-running cars.

Methanol can also be used as an additive for conventional gasoline, in a manner directly comparable to ethanol. When used in that manner, methanol will increase the volume of the gasoline (thereby requiring less gasoline to fill a tank), and it can also help reduce the air pollution from such cars.

Methanol is also a very useful feedstock for a wide variety of chemical manufacturing operations. In effect, the hydroxy group on methanol serves as a form of "handle", allowing methanol to be easily grabbed and manipulated by any number of other reagents, in ways that cannot be done with methane, a completely symmetric molecule that has no convenient handle.

For all of these reasons, enormous markets for methanol would quickly develop if "stranded" methane could be economically converted into methanol. Large-scale methanol creation, at remote oil or gas fields and other sources that generate waste or unwanted methane, would provide enormous commercial and job-creating benefits, by making efficient use of a valuable energy supply and chemical feedstock. It would also provide major environmental benefits, by eliminating the emissions of huge quantities of carbon dioxide into the atmosphere.

Even if the supply of gas at a very large oil field can justify construction and operation of a gas pipeline, the construction and operating expenses, environmental disruptions, and other costs and burdens created by that pipeline (which may include guarding it against potential terrorist attacks) could be avoided, if methanol conversion could be accomplished efficiently. As one example, the U.S. and Canadian governments, and various companies, must decide whether to build a gas pipeline, estimated to cost at least 15 billion dollars, from the North Slope of Alaska, through sensitive and fragile arctic regions in Alaska and Canada, to existing pipelines in southern Canada and the northern United States. However, the existing Alaskan oil pipeline (which travels almost directly south, to the port of Valdez on the southern coast of Alaska) is no longer running at capacity. It has been helping drain the giant Prudhoe Bay reservoir for nearly 30 years, and after that many years, that reservoir is approaching a state of depletion.

Therefore, the Alaska pipeline could handle both crude oil and methanol, by a well-known process in which alternating batches of crude oil and methanol would be pumped through a single pipeline. To prevent the crude oil from mixing with the methanol, alternating batches of different liquids can be separated from each other by mechanical plugs that can be sent through a pipeline. These types of plugs that travel inside pipelines are usually called "pigs", and they are widely used to clean and inspect the insides of oil pipelines.

Accordingly, if methanol could be efficiently converted into methanol at the already-existing Prudhoe Bay oil production facilities, it could eliminate the need to build a huge new gas pipeline through the arctic regions of Alaska and Canada. That would saving billions of dollars in costs, it would avoid major environmental disruptions in sensitive and fragile arctic areas, and it would sidestep and avoid a series of divisive, negative, and unhelpful political struggles and distractions.

Similarly, if methane could be efficiently and economically converted into methanol, it could be used as an energy-releasing fuel in various types of fuel cells. In general, the term "fuel cell" is used to refer to any type of reactor that uses controlled chemical reactions to release energy, while stopping short of burning the fuel in ways that create explosive bursts of energy (as occur in internal combustion engines).

Fuel cells that use methanol have never been commercialized on a large scale, since existing supplies of methanol could not justify large investments in research, development, or commercialization. However, if huge supplies of methanol could be made available by an efficient process that converts stranded methane into methanol, the research efforts that are currently being spent on attempts to create hydrogen-burning fuel cells, for automobiles, likely would be diverted to creating fuel cells that use methanol instead, since methanol offers a number of important advantages over hydrogen fuel. Those advantages include (i) a much higher energy content and density, in a tankful of methanol compared to a tankful of hydrogen; and (ii) greatly reduced requirements for extremely high pressures, which are required for hydrogen-burning fuel cells.

Despite all of these potentials, incentives, and opportunities, no one has previously been able to discover and create an efficient and economical method for converting methane into methanol, or into other useful and functional chemical derivatives. Despite at least 60 years of focused efforts by literally thousands of researchers at major universities and oil companies of all sizes, huge quantities of methane are still being wasted by burning in unproductive flares, or being pumped back into the ground.

A summary of prior efforts to convert methane into methanol is provided in numerous review articles (including Srivastava et al 1992, Fierro 1993, Crabtree 1995, and Labinger 1995) and books (e.g., Olah et al 1995). These efforts are also summarized in published PCT patent application wo 2004/041399, by the same Applicant herein. In addition, the Gas Utilization Research Forum has been formed by academic researchers and industrial managers, and a series of annual conferences, called "Monetizing Stranded Gas Reserves" (MSGR), is organized each year by a company called Zeus Development Corporation. Information can be obtained from the Internet website www.remotegasstrategies.com, which is run by Zeus Development Corporation. The agendas and speaker lists for those yearly conferences can be used to identify numerous companies, experts, and areas of focused research that are active in this field.

It must be understood that a major problem that has blocked all prior efforts to efficiently convert methane into methanol, in large commercial volumes, involves yield and selectivity. All prior proposed methods generated mixtures of different reaction products, which are not desirable in commercial systems. The goal of any efficient system is to produce a single desired product, in the highest possible quantities, and any byproducts must be regarded as unwelcome competitors, parasites, and problems, since they will decrease the yield of the desired product, and increase total costs of operation. Two issues are crucially important in assessing this factor. "Selectivity" refers to the extent to which a certain step (or a complete system) creates a single desired and intended product, with minimal quantities of competing and unwanted byproducts. "Yield" is related, and can be expressed in terms such as (i) percentages of reactants converted into intended products, or (ii) weight of products, divided by time (such as kilograms per minute, or tons per hour).

These issues are clearly discussed in Periana et al 1993, which described a strong desire to avoid creating any methyl radicals. As described by Periana, methyl radicals are more reactive than methane, and any methyl radical intermediates will tend to react, rapidly and in uncontrollable ways, with other reagents in the system, before those reagents can react in a desired way with methane. In Periana's words, "Radicals are among the most reactive species, and radical chemistry has been traditionally used for reaction with methane. These species can be generated under very forcing conditions or with very reactive reagents. However, under these conditions, the initial (and most useful) products of reaction are more reactive than methane, and selective reaction of methane in high yield is very difficult. Thus, reactions with oxygen can be accomplished but require temperatures above 700° C. Under these conditions, only low selectivities (<30%) to methanol have been reported at methane conversions above 10%, giving 3% overall yields [footnote 6]. Reactions with more reactive species such as chlorine can be carried out at 350° C, but the reaction selectivity to methyl chloride is still low and significant amounts of polychlorinated methanes are generated [footnote 7]. Given the low proton affinity and acidity of methane, it would not be expected that reaction of typical acids or bases with methane would occur at temperatures lower than those of radical processes. This unreactivity has been reported. Only with extremely reactive species, such as protons in 'superacid' media (SbF₅/HF) [antimony fluoride in hydrofluoric acid] are reactions with methane observed at lower temperatures [footnote 8]. However, these reactions are stoichiometric or use expensive reagents and thus are not practical for the large-scale oxidation of methane."

The 3% conversion figure cited in Periana et al 1993 can be placed in perspective by two additional comments. In his text, he stated, "The scientific and engineering community accepts as a general, conservative guideline that a high-selectivity (at least 85%), high-conversion (at least 30%) process for the oxidation of methane to methanol, with molecular oxygen as the final oxidant, could provide the basis for an economical process for the conversion of methane to a transportable material." Also, Periana's footnote 3 stated, "Achieving high selectivity at 30% conversion is much more challenging than at low (<5%) methane conversion because as product builds up with increasing conversion, it can become the preferred substrate for over-oxidation."

It also should be noted that various systems have been proposed that would use or create sulfuric acid or other sulfur compounds. These systems would generate large quantities of corrosive and hazardous byproducts and other potentially toxic wastes. Even if the byproducts or wastes can be recycled, they pose major obstacles to the economics and efficiency of any such system, especially when compared against the improved reaction systems disclosed herein.

One such effort was described in Basickes et al 1996, which described the testing of several "initiator" compounds to convert methane into a compound called methane-sulfonic acid, which has the formula H₃CSO₃H. Those initiator compounds included several metal salts, such as HgSO₄ (mercury sulfate), Ce(SO₄)₂ (cesium sulfate), and PdSO₄ (palladium sulfate), as well as the potassion salt of a compound called Marshall's acid. The formula for Marshall's acid can be written as H₂S₂O₈; the formula HO₃SO-OSO₃H indicates its structure as a symmetric di-acid with a peroxide (double-oxygen) linkage in the middle. The potassium salt is KO₃SO-OSO₃K.

One passage in Basickes et al 1996 is worth noting in particular. This passage postulates that two "Initiation" reactions are occurring, as follows:

K₂S₂O₈ ---> 2 KSO⁴*

KSO₄* + CH₄ ---> KSO₄H + *CH₃

It then postulates that two "Propagation" reactions are also occurring, as follows:

*CH₃ + SO₃ ---> CH₃SO₃*

CH₃SO₃* + CH₄ ---> CH₃SO₃H + *CH₃

That work, reported in Basickes et al 1996, was done by a research group headed by Prof. Ayusman Sen, at Penn State University. That line of research was later picked up by a research group headed by Prof. Alexis Bell, at the University of California, Berkeley, and it apparently is being commercialized by an Italian company, Atofina Chemicals Inc., as a method for commercial manufacture of MSA. The research reports on MSA production that have been published to date by Bell's group include Lobree and Bell 2001 and Mukhopadhyay and Bell 2002 (both of which used the potassium salt of Marshall's acid, K₂S₂O₈), as well as Mukhopadhyay and Bell 2003a and 2003b (which shifted to a system that evaluated a number of radical initiators, including K₂S₂O₈, K₄P₂O₈, H₂O₂, CaO₂, Br₂, Cl₂, and I₂, in the presence of metal chlorides such as calcium chloride, iron chloride, and especially rhodium chloride, RhCl₃).

Three important points should be noted about that work, to distinguish it from the subject invention.

First, the above cited articles, from Basickes et al 1996 through Mukhopadhyay and Bell 2003b, describe methods for making MSA, a valuable chemical used in electroplating, circuit board manufacturing, and manufacturing detergents. None of those items proposed any method for making methanol, which is much less valuable than MSA, as evidenced by comparing their prices. As of late October 2003, the price of methanol was about 22 cents per kilogram, from suppliers such as Methanex. By contrast, the price of MSA was roughly ten times higher, for the same weight. Accordingly, if a reaction scheme is designed to manufacture MSA as a product, it would be foolish to take the MSA and degrade it by breaking it apart to form methanol, which has only about a tenth of the value of MSA, per kilogram.

However, it must also be recognized that while there is only a small and limited market for MSA, there is an effectively limitless market for methanol, as both a chemical reagent, and as a clean-burning fuel (either by itself, or as a gasoline additive). Therefore, a reaction scheme that proceeds through MSA as merely one step in a series of intermediates, and which can then pass beyond the MSA to form methanol in huge quantities as a final product (while also allowing the sulfonic acid group to be endlessly recycled, as more methane is continuously passed through the system and converted into methanol) is a very different reaction system, with an entirely different purpose.

A second major difference between the MSA production methods discussed above (initially by Ayusman Sen's research group, then by Alexis Bell's research group), compared to the current invention, involves their use of salt compounds as radical initiators, in acidic media. It is believed and suspected by the Applicant herein that salt compounds (such as the potassium salt of Marshall's acid), if used in acidic media, cannot perform effectively in the reaction systems disclosed herein.

The reasons for this are believed to involve at least three factors, which are complex but which can be summarized as follows. First, when Marshall's acid anions (S₂O₈²⁻) are released by a potassium salt (or other salt) of Marshall's acid, in an acidic medium that contains a large quantity of H⁺ protons, those anions and protons will reach equilibrium concentrations of various ionic species, including certain ionic compounds that are likely to quench and terminate the radical-initiated chain-reaction mechanisms that are involved in this current invention.

The second factor relates to the tendency of metal ions (such as potassium ions) to interfere with certain reactions that are involved in the current invention.

The third factor, also addressed below in a discussion of unwanted byproducts and wastes, is that salt compounds tend to generate unwanted byproducts, including (in many cases) layers of crystalline deposits that accumulate in pipes and vessels, impeding flow rates, heat transfer, etc.

Because of those and other factors, research using reagents such as salts of Marshall's acid is done solely in small-scale tests in academic research, rather than in commercially-oriented industrial research. A direct comment on the industrial potential of reactions that use K₂S₂O₈ as a radical initiator was offered by a researcher who works for Shell International Exploration and Production, a subsidiary of one of the world's largest and most successful oil companies. In an article entitled, "A Chemical Alternative to Natural Gas Flaring," that researcher and a coauthor bluntly stated, "At any rate, the reaction would only be of industrial interest with an aerobic oxidant. For that reason we never examined what happened to K₂S₂O₈, which one would never use in an industrial process." (Golombok et al 2003).

For these and other reasons, the Applicant chose to pursue the use of "free acid" peroxo-acids, such as Caro's acid or Marshall's acid, rather than using salts of those acids. Subsequently, this hunch by the Applicant was confirmed by direct experimental evidence, which used parallel tests under identical conditions to show that the potassium salt of Marshall's acid was ineffective in triggering the reactions disclosed herein, while the free acid form of Marshall's acid was highly effective and selective. Those laboratory tests were carried out by a skilled expert in this field of research at his own initiative, since he believed a K₂S₂O₈ system would work until he saw the actual experimental evidence to the contrary.

A third major difference between the MSA production methods of the prior art, and the reagents and methods of the current invention, further reveals why the reaction systems discovered by Sen's research group, and refined by Bell's research group, were recognized as being well-suited for making limited quantities of MSA, but were not suited for making huge quantities of methanol. This factor involves the waste products generated as a byproduct of the potassium salts and other reagents tested by the Sen and Bell research groups. For each kilogram of MSA created from methane, those reaction schemes produce substantial quantities of acids and salts, as unavoidable and generally corrosive, toxic, and/or fouling wastes.

By contrast, the reaction scheme disclosed herein is highly efficient and economical, in large part because it uses a combination of chain reactions and recycling steps that generate a remarkably small and easily manageable quantity of undesired chemical byproducts and waste.

On a theoretical basis, if the chain reactions can continue indefinitely, this system can produce absolutely no waste products at all. However, since no chain reaction system is perfect or can continue forever, it is presumed that: (i) a small "makeup" quantity of a radical initiator should be added continuously to the system, presumably by means such as spraying a fine mist of liquid droplets into a fast-moving gas stream, in order to keep the selectivities and yields of the desired reactions as high as possible; and (ii) a waste product (which most likely will comprise a mixture of various chemicals) will gradually accumulate, in proportion to the small quantities of radical initiators that will need to be continually added to the system to keep it running under optimal conditions.

This type of waste generation, at very low levels as a result solely of chain reaction limitations, is expected to be orders of magnitude less than the generation of acidic and salt wastes that are generated as unavoidable byproducts of the reaction systems that use potassium salts and other radical initiators, as disclosed in the above-cited papers that include Basickes et al 1996 through Mukhopadhyay and Bell 2003b.

For completeness, it should be noted that other efforts to convert methane into functional derivatives have used other approaches that do not involve sulfur. As examples, US patents 3,979,470 (Firnhaber et al 1976), 4,523,040 (Olah 1985), 4,804,797 (Minet et al 1989), and 6,452,058 (Schweitzer et al 2002, assigned to Dow Global Technologies) describe halogenation processes that can create methyl chloride, methyl fluoride, methyl bromide, or other methane derivatives that contain halogen atoms. These systems each have their own types of valuable but limited industrial utility, in forming halogenated chemicals; however, they are not suited for creating a commodity chemical such as methanol, which has effectively unlimited markets both as a reagent, and as a fuel source and gasoline additive.

Despite many efforts over more than half a century, no one ever previously disclosed a reaction scheme that was efficient enough to justify widespread commercial use, for creating methanol from stranded methane. Instead, at thousands of oil and coal fields around the world that are not served by gas pipelines, companies that produce oil or coal continue to burn huge quantities of methane in unproductive flares (aggravating global warming, by pumping huge quantities of carbon dioxide into the air), and they pump huge quantities of methane back into the ground. Multinational oil companies are planning to spend many billions of dollars to create liquified natural gas (LNG) facilities and fleets, even though LNG processing bums about half of the methane, to drive the refrigeration and heating processes. In addition, companies and politicians in the U.S. and Canada are wrestling with very difficult and divisive decisions about whether to build a huge and extremely expensive gas pipeline through fragile arctic regions in Canada, because they know of no way to convert that gas into methanol, which could be shipped easily through the existing oil pipeline that already crosses Alaska.

These and other huge problems could be avoided entirely, and solved easily, if an efficient and economical method became available for converting methane gas into methanol.

Accordingly, one object of this invention is to disclose a reaction system that can be useful in the pursuit of converting methane into methanol, in a more efficient, more selective, and less expensive manner than any previously known reaction system.

Another object of this invention is to generate only very small quantities of waste or unwanted byproducts.

These and other objects of the invention will become more apparent through the following summary, description, and figures.

### SUMMARY OF THE INVENTION

In view of the above, the present invention provides a method according to claim 1. Advantageousembodiments may be implemented according to any of claims 2-4. While the scope of the invention is defined solely by the claims, reagents and methods with low thermodynamic barriers are disclosed, for converting small hydrocarbons such as methane into oxygenated or other intermediates or products, such as methanol. This reaction system uses a small quantity of a radical initiator, such as Marshall's acid, which can be generated on-site and then split into radicals by mild heating. These radicals will remove a hydrogen atom (both a proton and a nucleus) from methane, to generate methyl radicals (H₃C*) and a small quantity of sulfuric acid. Sulfur trioxide (SO₃) is added, and the methyl radicals will combine with it to form methyl sulfonate radicals. Additional methane is added, and the methyl sulfonate radicals will attack it, to form methanesulfonic acid (MSA, H₃C-SO₃H), in a reaction that will regenerate a renewed supply of methyl radicals (H₃C*). This allows the process to be continued, in a chain reaction, by continuously adding more sulfur trioxide and methane. The MSA can be removed and sold as a product, or it can be heated, to split it into methanol and sulfur dioxide. The sulfur dioxide can be regenerated into sulfur trioxide, which can be fed back into the system. The methanol can be condensed, and transported to market in liquid form via pipelines, tankers, etc. Since the process generates only very small quantities of byproducts (mainly sulfuric acid from the Marshall's acid), this system can be used to convert huge quantities of stranded and wasted methane gas, from oil or coal production facilities and other sources, into useful products that can serve unlimited markets.

Methods and reagents are also disclosed for using bi-functional reagents (such as a bromate sulfate compound, HO₃S-O-BrO₂) with electrophilic and nucleophilic domains positioned adjacent to each other in the same molecule. Such reagents can create coordinated proton and electron shifts in methane or other hydrocarbons, using symphoric, anchimeric, or other "neighboring group" effects, to create transitional intermediates with reduced thermodynamic barriers. This can improve the selectivity and yield of reaction systems for converting methane or other lower alkanes into methanol or other valuable intermediates or products.

### BRIEF DESCRIPTION OF THE DRAWINGS

FIGURE 1 depicts a chemical reaction scheme that uses hydrogen peroxide and sulfur trioxide to create Marshall's acid, H₂S₂O₈, which is mildly heated to break the peroxide bond. This releases radicals, HO₃SO*, which will "activate" methane by taking away a hydrogen atom, converting the methane (CH₄) into a methyl radical (H₃C*).
FIGURE 2 depicts a chemical reaction that uses methyl radicals (H₃C*) and sulfur trioxide, to form methanesulfonic acid (MSA), by means of a multi-step process that will also create a new methyl radical. This establishes a chain reaction, and the newly created methyl radicals will react with newly-added SO₃. The stable MSA can be removed from the vessel by condensation. It can be sold as a product, used as a reagent, or "cracked" to release methanol, and sulfur dioxide which can be regenerated back into sulfur trioxide.
FIGURE 3 depicts a set of reactor vessels that can be used to convert methane into methanol, using the Marshall's acid pathway.
FIGURE 4 depicts two potential unwanted side reactions, involving free radicals, that might pose concerns for a radical initiator system as shown in FIG. 1. However, computer modeling indicates that either of these two possible reactions will simply return to the desired state, by re-forming the desired reactants, rather than degenerating into unwanted byproducts.
FIGURE 5 depicts a reaction system using a bi-functional bromate-sulfate reagent that can activate lower alkanes such as methane, by using coordinated proton and electron shifts to create intermediates with low thermodynamic barriers.
FIGURE 6 depicts the energy profile of a pathway for converting methane to methanol using the bromate-sulfate reagent, compared to an iodine system of the prior art.

### DETAILED DESCRIPTION

As briefly summarized above, reagents and methods are disclosed for converting small hydrocarbons (such as methane) into oxygenated or other intermediates or products (such as methanol or methanesulfonic acid).

The pathways that are provided by these reagents and methods offer several important advantages. These will be briefly listed, then they will be discussed and illustrated in more detail by using an exemplary system that uses Marshall's acid as a radical initiator, to trigger a chain reaction that uses methyl radicals to convert methane into methanol, in large quantities, without generating any substantial quantities of waste.

The advantages offered by this system include:
1. These pathways have low thermodynamic barriers. This includes low "heat of activation" levels, and low or negative "Gibbs free energy" pathways.
2. As a direct result of their low thermodynamic barriers, these reactions can be carried out at relatively low and mild temperature and pressure combinations, and will result in relatively high efficiency, selectivity, and yield, all of which are highly valuable.
3. These pathways can generate large quantities of product without also generating large quantities of waste, by using a small quantity of a "radical initiator" compound to set a chain reaction in motion, and then allowing the chain reaction to sustain the process, while adding only small "makeup" quantities of the radical initiator.
4. These pathways allow the endless regeneration and recycling of all sulfur compounds that are used or produced by the system. In specific, sulfur trioxide (SO₃) is pumped into the reactor vessel. It reacts with methyl radicals to form methanesulfonic acid (MSA) radicals, which then react with more methane to form stabilized MSA. The MSA is removed from the reactor, and can be "cracked" to release methanol (the main product) and sulfur dioxide. The sulfur dioxide can be regenerated back into sulfur trioxide, and the regenerated sulfur trioxide can be pumped directly back into the reactor vessel. This cycle (SO₃ to MSA to SO₂, then back to SO₃ again) can be repeated an endless number of times, without generating any sulfuric acid or other acidic or toxic wastes.
5. These pathways are run in an entirely anhydrous manner, which does not use or produce water, and these pathways also avoid any use of any salts. This anhydrous, non-salt approach offers numerous advantages; among other things, it makes the system more efficient, less corrosive, and less subject to fouling by mineral deposits inside the vessels or pipes, and it reduces the formation of unwanted byproducts and wastes.

These factors and advantages can be better understood by considering an exemplary reaction system, described below.

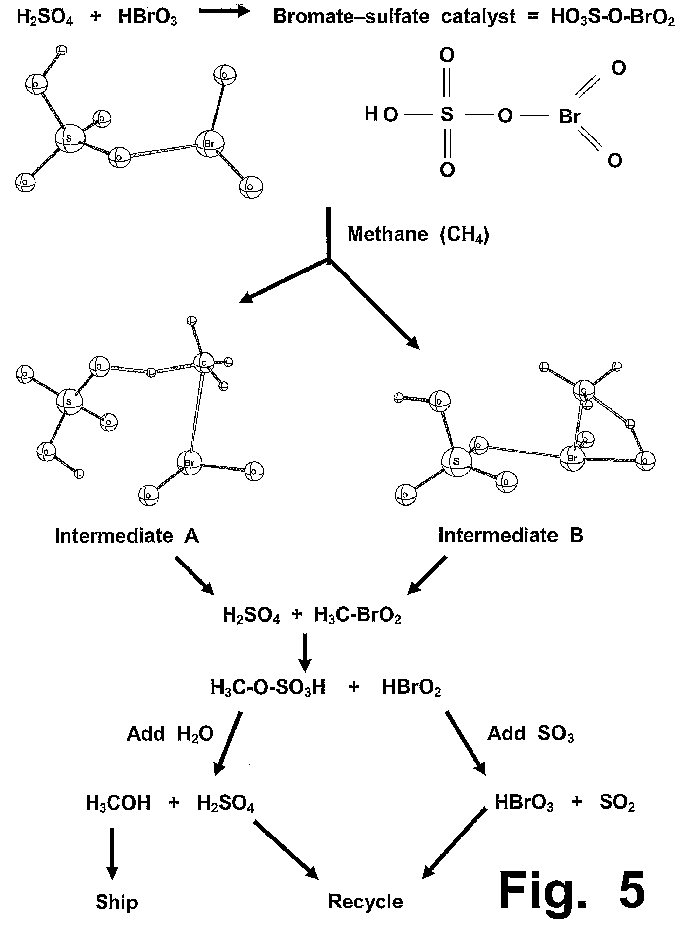

Published Patent Cooperation Treaty application WO 2004/041399 describes the sequence of efforts, insights, and realizations that led to the system described herein. Briefly, the Applicant initially began studying reagents that can use symphoric, anchimeric, or other "neighboring group" effects to exert two different effects on a methane molecule. That work led to a bromate-sulfate system that creates methyl bisulfate, H₃COSO₃H, with an oxygen atom between the carbon and sulfur atoms, as shown in FIG. 5. The Applicant began studying ways to convert that system to anhydrous processing, to avoid water and sulfuric acid, and he encountered a reference to an old British patent from the 1940's, GB 632,820. That led him to three corresponding US patents (2,493,038; 2,492,983; and 2,553,576) coinvented by John Snyder and Aristid Grosse. Those patents described the synthesis of compounds with direct carbon-sulfur bonds, with no oxygen in the middle. Additional research led him to Basickes et al 1996, and to Mukhopadhyay and Bell 2002 and 2003a, which used the potassium salt of Marshall's acid.

Since the Applicant wanted to avoid salts and water, he began considering the free (non-salt) version of Caro's acid as a radical initiator, and then Marshall's acid after he located US patent 3,927,189 (Jayawant 1975, assigned to DuPont). With the help of a graduate student who was skilled in chemical modeling, using the Amsterdam Density Functional program (release 2.3.3, by Scientific Computation and Modelling (www.scm.com, described in articles such as te Velde et al 2001), the Applicant decided to model a possible approach using Marshall's acid, broken apart to release radicals, to trigger a chain reaction that could use methyl radicals. The results indicated that this system is feasible and practical, with surprisingly high yield and selectivity. The Applicant disclosed those results in confidence to certain selected people, including Prof. Ayusman Sen at Penn State University, whose research group did the laboratory tests described in the Examples. The results of those tests confirmed that this system does indeed work in the manner intended.

That series of steps and insights is described in more detail in PCT application WO 2004/041399.

### CONVERSION OF METHANE INTO METHANESULFONIC ACID (MSA)

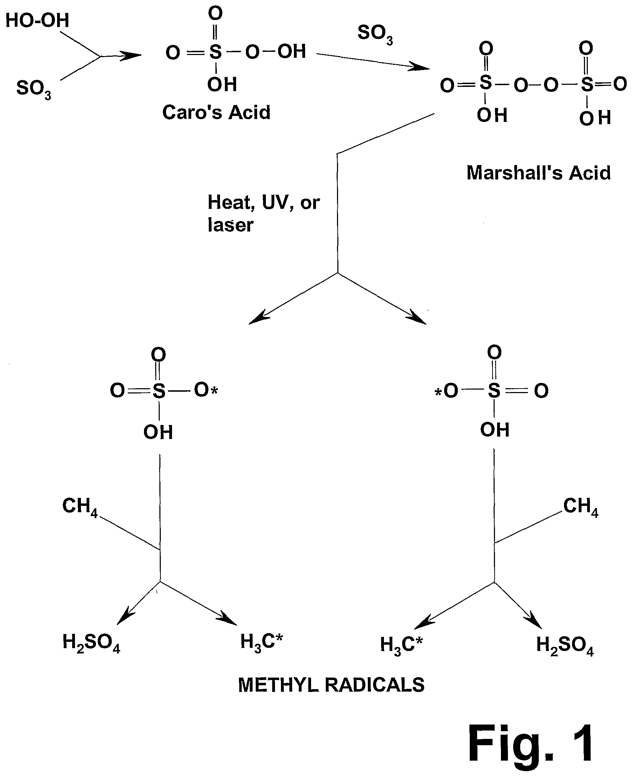

A preferred embodiment of this invention, referred to herein as "the Marshall's acid system" uses a small quantity of a compound called Marshall's acid to initiate a chain reaction, involving the conversion of methane into "activated" methyl radicals. This part of the process is illustrated in FIG. 1.

As shown near the top of FIG. 1, the manufacture of Marshall's acid is carried out by reacting hydrogen peroxide (HOOH) with sulfur trioxide (SO₃), to form a compound called peroxy-monosulfuric acid, which has the common name Caro's acid. Reactor vessels and preferred conditions for carrying out this reaction are disclosed in US patents 2,789,954 (Fell, 1957), 3,900,555 and 3,939,072 (Jourdan-Laforte, 1975 and 1976), and 5,304,360 (Lane et al).

Additional SO₃ is then reacted with the Caro's acid, to convert it into peroxy-disulfuric acid, which has the common name Marshall's acid. This reaction can be carried out as disclosed in US patent 3,927,189 (Jayawant 1975, assigned to DuPont). The '189 patent, which disclosed methods for creating Marshall's acid in a relatively stable form that could be stored for some period of time, indicated that the reaction should be done under mild conditions, with temperatures not to exceed 45°C, since higher temperatures would lead to more rapid decomposition of the resulting product.

However, the goal of manufacturing Marshall's acid for the purposes herein is not to create a stable and storable product, but instead to create a compound that will immediately be split in half, to release free radicals that will then react with methane, to strip hydrogen atoms away from the methane. Accordingly, certain precautions described in the '189 patent will not come into play here, and various modifications of those procedures (such as the use of higher reaction temperatures, which may include simply omitting any efforts to remove heat from the reaction vessel, since heat will be released by the exothermic creation of Marshall's acid) should be tested and evaluated, since they may be able to speed up and/or increase the efficiencies and yields of reactions used to create Marshall's acid.

If Marshall's acid is used in large-scale industrial facilities for converting huge quantities of methane into methanol or other products, various methods and devices are likely to be developed for improving its methods of on-site manufacture followed by immediate use. In particular, higher-than-normal temperature and pressure combinations should be evaluated, which are designed to lead, not to the formation of cool and stable Caro's acid, but to the rapid conversion of Caro's acid into Marshall's acid, preferably at temperatures that will help promote the breakage Marshall's acid, to release HSO₄* radicals.

Regardless of whether any such improved methods are developed, Marshall's acid is a well-known compound, and methods have already been disclosed for making it in commercial quantities (in particular, US patent 3,927,189, Jayawant 1975, is entirely devoted to a process for manufacturing Marshall's acid). Therefore, the manufacture of Marshall's acid can be carried out using known equipment and methods.

After Marshall's acid has been created, it is then split into two equal and identical halves, by breaking the peroxide bond. This can be done by mild heating; alternately, it may be possible to accomplish it even more rapidly and controllably by other means, such as ultraviolet radiation, or by using radiation having a specific wavelength, from a "tuned" laser (see, e.g., US patent 4,469,574, Keehn et al 1984).

The resulting Marshall's acid radicals (HO₃SO*) are highly unstable, and highly reactive, due to the presence of an unpaired electron on one of their oxygen atoms. This makes them well-suited for use as "radical initiators" in the reactions disclosed herein. When mixed with methane, a Marshall's acid radical will extract a complete hydrogen atom (both a proton, and an electron) from the methane. This transfer of a hydrogen atom converts a Marshall's acid radical into stable sulfuric acid (HSO₄), and it generates a methyl radical, H₃C*.

As shown in FIG. 1, each mole of Marshall's acid will release two "strong" free radicals that are identical to each other, rather than just one "strong" radical as created by Caro's acid and most other radical initiators. The reference to "strong" free radicals includes free radicals with a highly electronegative inorganic atom, such as HS₃SO*; and which have sufficient "reactivity" to quickly and efficiently remove a hydrogen atom from methane. "Strong" radicals do not include simple hydroxy (HO*) radicals, as released by hydrogen peroxide or Caro's acid, since hydroxy radicals are not strong enough to quickly and effectively remove a hydrogen atom from methane under processing conditions that are likely to be preferred for use herein.

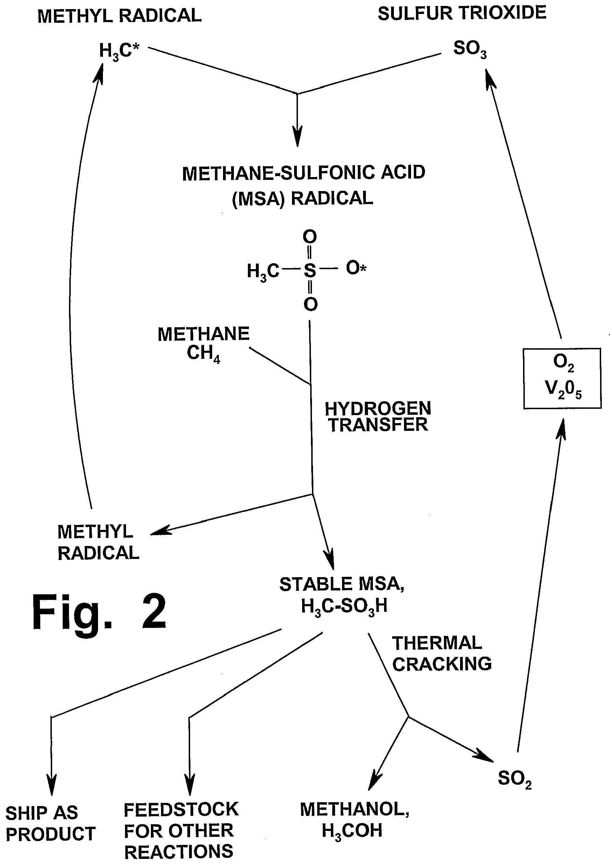

After a set of methyl radicals has been created by a radical initiator, the methyl radicals will then launch a chain reaction, as illustrated in FIG. 2, which leads to the formation of methanesulfonic acid (MSA). This process requires a multi-step sequence. A selected oxidant compound, such as sulfur trioxide (SO₃), is pumped into the reactor, and the methyl radicals will combine with it, to form methyl sulfonate radicals. As more methane gas is added, the methyl sulfonate radicals will attack the added methane, and remove a single hydrogen atom from each methane molecule, thereby creating a new methyl radical.

This process allows MSA radicals to create complete and stabilized molecules of MSA, which can then be removed from the system, for further processing.

At the same time, that hydrogen transfer also generates a renewed supply of methyl radicals (H₃C*). Accordingly, this series of reactions allows the process to be continued and sustained, as a chain reaction. As long as more sulfur trioxide and methane continue to be added to the system, they can continue to form MSA.

Because of certain types of electron shifts, a methyl radical (H₃C*) that has already lost one hydrogen atom will not readily or easily give up a second hydrogen atom. This is an important feature of this invention, and it directly contrasts with certain other chemical reactions involving methane. For example, when methane is treated with a halogen such as chlorine, the loss and displacement of a first hydrogen atom tends to enable or even accelerate the loss of additional hydrogen atoms, thereby leading to mixtures of carbon dichloride, trichloride, and tetrachloride. However, the opposite happens when methane loses a hydrogen atom and becomes a radical. That realization was another crucially important insight, which helped the Applicant develop the reaction scheme disclosed herein, in a way that can lead to a single product with good selectivity, rather than being pulled in four different directions, once the reaction commenced.

As mentioned above, MSA can be removed from the reactor vessel, by using conventional condensation devices. After removal from the reactor, any of several things can be done with it, as indicated in FIG. 2.

One option is to sell the MSA, as a valuable product in its own right. Another option is to use the MSA as a feedstock in any of various other chemical processes.

However, the market for MSA, and the known uses for MSA, are both limited. They are believed to amount to only a few tens of millions of dollars per year, worldwide, while literally billions of dollars worth of methane are being burned and wasted in flares, or reinjected back into the ground, every month.

Therefore, the larger value of this system comes from the fact that MSA can be "cracked" to release methanol and sulfur dioxide. This cracking can be done by thermolysis (also called pyrolysis) at elevated temperatures, as described in US 2,553,576 by Grosse and Snyder, who used silver carbonate as a catalyst, and refluxing temperatures between 300 and 350°C.

Other catalysts, temperature ranges, and operating parameters can be evaluated if desired, and if worldwide supplies of MSA suddenly increase by orders of magnitude, due to its synthesis from stranded methane, it is extremely likely that improved methods and refinements for cracking MSA into smaller compounds can and will be developed, including various methods that will be patentable in their own right.

The sulfur dioxide which is released by cracking MSA can be regenerated back into SO₃, by contacting it with oxygen (O₂, which can be obtained in purified form, from air), in the presence of a catalyst such as vanadium pentaoxide, V₂O₅. The regenerated SO₃ can be pumped directly back into the reactor vessel, without creating any waste, to keep the process running.

Methanol which is released from MSA by thermal cracking has a virtually unlimited market; among other things, it can be used as a chemical feedstock, as a clean-burning fuel, and as a gasoline additive that can reduce air pollution from automobiles, and that also can reduce their consumption of gasoline. As a compound that remains in liquid form even at room temperatures and low pressures, it can be pumped through pipelines, and loaded into tanks, tanker trucks, and ocean-going tanker ships, for delivery to commercial markets.

Since the process generates only very small quantities of byproducts (mainly a small quantity of sulfuric acid, from the small quantity of Marshall's acid that is added to the system in limited "makeup" volumes) this system can be used to convert huge quantities of stranded and wasted methane gas, from oil or coal production facilities and other sources, into useful products that can serve unlimited markets.

On the subject of "makeup" volumes of Marshall's acid, no chain reaction can ever reach an ideal and theoretical 100% yields, and there are always minor losses, inside any reactor vessel in the real world. As just one example, if two methyl radical happen to collide and react, they will simply form ethane, C₂H₆, which is a stable lower alkane. This will terminate both of the radicals, and they will no longer be reactive.

Because of these and other losses and chain terminations, it will be necessary to introduce, during the reaction (and preferably in a continuous manner) a relatively small quantity of free radicals, from Marshall's acid or another radical initiator, into the system. This typically can be done by injecting a fine mist of a liquid, into a reactor vessel.

Accordingly, a method for converting methane into an oxygenated derivative may comprise the following steps:
a. removing hydrogen atoms from methane, thereby generating methyl radical intermediates, each having an unpaired electron, within a reactor device;
b. contacting the methyl radical intermediates with a selected oxide compound (such as sulfur trioxide, in the system shown), under conditions that cause the methyl radicals to react with the selected oxide compound in a manner that forms a methylated oxide radical, wherein the methylated oxide radical has sufficient reactivity to remove hydrogen atoms from newly-added methane; and,
c. reacting the methylated oxide radical with methane, under conditions that cause removal of hydrogen atoms from the methane, thereby forming stabilized methylated oxide molecules while also generating newly-formed methyl radicals.

Stated in alternate terms, this system comprises a method for converting a lower alkane into an oxygenated derivative, comprising the following steps:
a. removing hydrogen atoms from at least one lower alkane compound, thereby generating alkane radicals;
b. contacting the alkane radicals with a selected oxide compound under conditions that will cause the alkane radicals to bond to the selected oxide compound, thereby forming alkylated oxide radicals;
c. reacting the alkylated oxide radicals with at least one lower alkane, under conditions that cause removal of hydrogen atoms from the lower alkane, thereby forming stabilized alkylated oxide molecules while also generating newly-formed alkane radicals.

### Manufacturing System (Plant Layout)

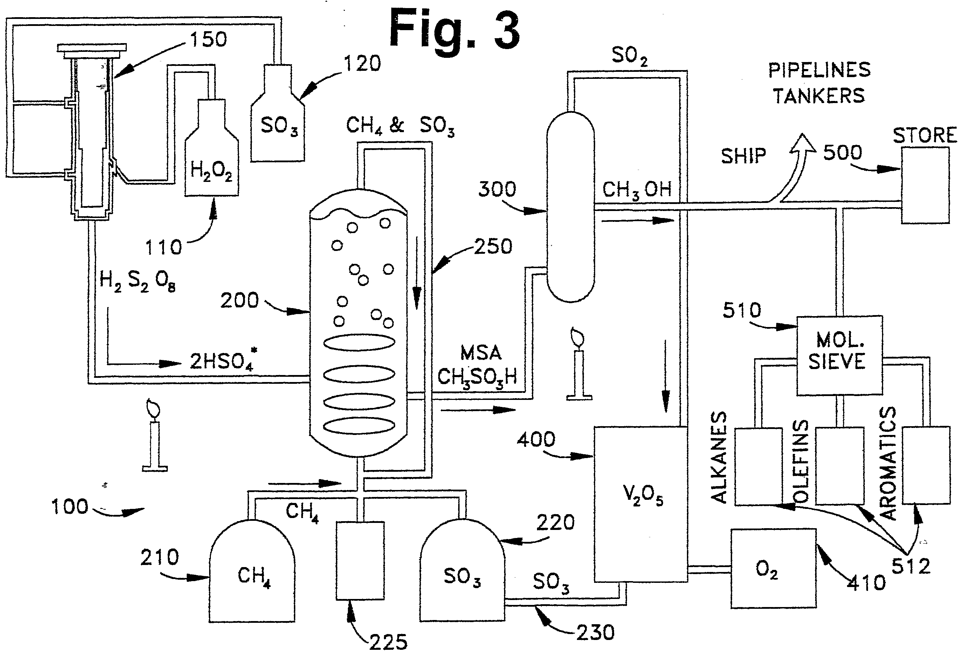

FIG. 3 provides a schematic layout of a manufacturing system 100 (often called a "plant" in the petrochemical industry) that can be used to carry out the reactions of this invention. Starting near the upper left, reagent supply container 110 contains hydrogen peroxide, H₂O₂. Reagent supply container 120 contains stabilized anhydrous liquid SO₃, or an alternate sulfonating agent that can be converted into Caro's acid and/or Marshall's acid. Both of these reagents are pumped into a suitable acid formation vessel 150, where they will combine and react to initially form Caro's acid, and preferably to then form Marshall's acid, H₂S₂O₈, in a second stage of the sulfonation reaction. Acid formation vessel 150 is modeled after a similar vessel having annular reaction zones, shown in US patent 5,304,360, and used to create Caro's acid; it has been modified by the addition of a separate and additional inlet, to allow additional SO₃ to be added to the vessel, to convert the Caro's acid to Marshall's acid.

The Marshall's acid will emerge from the bottom of acid formation vessel 150, and it will be heated, subjected to UV or laser radiation, or otherwise treated, to split it into HSO₄* free radicals, as shown in FIG. 1. These free radicals will be pumped, presumably in the form of a fine mist, entrained liquid, etc., into a main reactor vessel 200, which preferably should contain internal baffles, agitators, and/or other structures that will promote high levels of liquid/gas contact and interaction.

Main reactor vessel 200 will be receiving a steady supply of both methane and SO₃, from supply tanks 210 and 220 (via pump 225), and also from one or more recycling conduits 250 that will collect any unreacted methane or SO₃ that emerge from reactor 200. In most facilities that will deal with large volumes of methane that has been separated from crude oil at an oil field, the methane supply pump 210 presumably will receive its supply of methane gas from a storage or surge tank that receives and holds semi-pressurized methane gas, after the gas has been removed from crude oil in a separation vessel.

In cases in which sulfonated products are not removed and sold, the SO₃ supply will be continuously recycled; therefore, the "makeup" volumes that will be required to replace small and gradual losses will not be nearly as large as the volumes of methane that will be processed.

However, it also should be recognized that methanesulfonic acid (MSA, CH₃SO₃H) is a valuable and useful chemical product in its own right; indeed, it is worth roughly 10 times more than methanol, on an equal-weight basis. Therefore, it can be sold as a valuable product, or used as a valuable chemical feedstock.

It should be noted that the much higher value for MSA, compared to methanol, can help explain why the results seen by Sen's group and Bell's group, involving methods for manufacturing MSA by using K₂S₂O₈, apparently were not recognized as suggesting potential pathways that might offer an economical method for manufacturing methanol. As mentioned above, methanol has only about 1/10th the value of MSA.

Nevertheless, now that this new approach to manufacturing methanol has been disclosed, it should be recognized that it also discloses a valuable new method for creating MSA, as a product, rather than just as an intermediate. Therefore, if desired, MSA (or various other sulfonated products or intermediates, if additional processing is carried out) can be removed directly from the new system disclosed herein, simply by sending some or all of the MSA that leaves the main reactor vessel 200 to a storage tank, rather than to a heating and cracking vessel 300 that will break the MSA into methanol and SO₂. It should also be recognized that if MSA or any other sulfonated products are removed from the system as products, the supplies of SO₃ that must be fed into the system also will need to be increased in a corresponding manner.

Any known method or machine for increasing the contact and interactions between the reagents inside the main reactor vessel 200 can be evaluated, using routine experiments, to determine their suitability for use as disclosed herein. For example, methane and SO₃ from supply pumps 210 and 220 might be pre-mixed, before they enter reactor vessel 200; alternately, they might be introduced in a counterflow manner, by introducing gaseous methane into the bottom of vessel 200, so that it will bubble and rise upward, while liquid SO₃ is pumped into the top of vessel 200 so that it will flow downward due to gravity. Similarly, any known or hereafter-discovered system, type, or combination of baffles, trays, meshes, fluidized particulate bed reactors, rotating bed reactors, SO₃-coated particulates, and other devices, methods, or formulations can be evaluated for use as disclosed herein, to determine whether they can improve the yields of the reactions disclosed herein.

In particular, one class of candidate reactor vessels that may be well-suited for such use includes spinning-bed reactors, as described in US patent 4,283,255 (Ramshaw et al 1981, assigned to Imperial Chemicals), and US patent 6,048,513 (Quarderer et al 2000, assigned to Dow Chemical Company). These devices normally use a fairly wide and thick disk that spins at a high speed, thereby acting as a centrifuge that drives gases and liquids from a center input point, toward the outside of the bed. They often use porous metallic mesh as the media. The wires that form the mesh can be made of stainless steel or other relatively strong and inexpensive material, which are coated with a thin layer of a more expensive catalyst, such as a vanadium oxide, by means of electroplating, sputter-coating, or other means.

Another class of candidate reactor vessels that may be well-suited for such use includes "loop" reactors, as described in US patent 5,159,092 (Leuteritz 1992, assigned to Buss AG of Switzerland). These are often referred to as Buss (pronounced "boose") reactors. A subcategory of loop reactors that also deserves attention comprises "monolithic" loop reactors, as described in Broekhuis et al 2001. Loop reactors typically use a combination of (i) a main reactor vessel, which contains an ejector mixing nozzle, a solid catalyst bed, or some other device that cannot be removed from the main vessel; and, (ii) a separate and typically smaller "secondary" vessel, which receives a liquid or gas that has been removed from the main vessel, and which treats that liquid or gas by some chosen means (such as by a heat exchange process) before returning it to the main reactor vessel. In this manner, the secondary device (along with its piping and pumps, which form a loop that is connected at both ends to the main vessel) can be used to help control and regulate what is occurring inside the main vessel, without disrupting a catalyst bed or other system or device that is operating inside the main vessel.

It also should be recognized that various types of solvents can and should be evaluated for running the system disclosed herein. Stabilized liquid sulfur trioxide, methanesulfonic acid, or an MSA-sulfur trioxide mixture offer promising candidates for evaluation as liquid media that can allow everything to keep running smoothly and efficiently inside the main reactor vessel 200, since each of those compounds will already be present in the reactor vessel, as either reagents or products. Alternately, any other type of candidate solvent or liquid media that may be of interest can be computer-modeled at very low cost, and those that appear to be interesting based on such computer modeling can be tested, in scaleup or pilot plants.

The MSA (CH₃SO₃H) that is generated within the main reactor vessel 200 will be collected (such as by using condensate traps), and pumped to a separate heating or "cracking" vessel 300, where it will be heated to cause it to break apart into methanol (CH₃OH) and sulfur dioxide (SO₂). If desired, the breakdown reaction inside cracking vessel 300 can be catalyzed or promoted in any suitable manner, such as by using a platinum or other metal catalyst. This type of reaction, in which a larger molecule is broken into smaller fragments (without the addition of water components into either of the smaller molecules) can be referred to as pyrolysis, thermolysis, "cracking", or similar terms.

Methanol, here the desired product, generally will be pumped into a collection or holding tank, shown as tank 500 in FIG. 3, if substantial volumes are involved, for subsequent pumping into a pipeline, tanker truck or ship, nearby factory, etc. Depending on various factors (including the purity of the methane stream that is being processed, as well as reaction parameters inside vessels 200 and 300), other organic compounds may be entrained in the methanol stream that emerges from heating vessel 300. Such impurities may include lower alkanes or alkane derivatives, olefins, alkenes, or other unsaturated compounds or derivatives, and benzene or other aromatic compounds. If desired, these can be separated out from the methanol stream, and collected for sale or use as valuable products in their own right. This type of separation can be carried out by, for example, a reactor bed 510 that contains a "Zeolite" (aluminosilicate) compound such as "ZSM-5", sold by the ExxonMobil Corporation. This material functions in a manner comparable to a molecular sieve, by separating different components from a mixed organic liquid stream.This can allow various separated products to be divided among different collection tanks 512 (which can receive and hold, for example, batches that have been separated into major categories, such as alkanes, olefins, and aromatics as shown in FIG. 3, or which can hold different batches that have been separated based on any other criteria).

Gaseous SO₂ also will emerge from the heating vessel 300. It can be passed through a suitable reactor 400, which will also receive oxygen (O₂) from a separate oxygen supply vessel 410, to oxidize the SO₂ to its higher oxidation state, SO₃. Reactor 400 preferably should contain a catalyst, such as vanadium pentaoxide (V₂O₅), to facilitate reaction of the SO₂ and the O₂ to form SO₃. The SO₃ will be returned to the main reactor vessel 200.

During benchtop analysis and testing or other small-scale use of these reactions, the oxygen supply vessel 410 can use bottled oxygen, or any other available source. In large-scale manufacturing operations, the oxygen supply vessel 410 preferably should use a device that can extract O₂ directly from the atmosphere, such as a so-called "pressure swing absorber" (PSA) system, sold by companies such as IGS, Holtec, and other manufacturers known to those skilled in the art.

The handling and utilization of SO₃ can involve any known or hereafter-discovered method that will enhance the efficiency of the operations disclosed herein, or that will otherwise improve these operations in any other significant manner (such as by reducing waste products, etc.). Numerous methods and reagents which facilitate various aspects of generating or handling SO₃ are known, including (for example):
(i) the use of various derivatives of boron, phosphorous, or sulfur to stabilize SO₃ in liquid form, as described in articles such as Gilbert 1965; and,
(ii) the use of solid supports (which can be in the form of small particles, to allow pumping and handling inside a fluidized or constrained "bed", column, or other device), to create relatively thin layers of liquid SO₃ that will coat the surfaces of the particles.

Any such method or reagent, and any combination of such methods or reagents, can be evaluated to determine their suitability for use as disclosed herein.

### CALCULATED ENTHALPY AND FREE ENERGY VALUES

As known to anyone who works with computer modeling of chemical reactions, certain numerical values that can be calculated by computers can indicate of how rapidly and efficiently a proposed pathway will be followed. One of those values is usually called Δ-H. The Greek symbol Δ-(called "delta", in English) indicates that a numerical value for Δ-H indicates the difference between two energy states, rather than an absolute value. In chemical terms, "H" refers to something that can be regarded as heat; however, this type of heat refers to heat potential stored in the form of chemical bonds, rather than the current temperature of a particular compound or material. To distinguish this quantity from temperature, it is usually called *enthalpy* by chemists.

For many classes of chemical reactions, two different Δ-H (enthalpy) values must be calculated, and both are crucially important. One value, called the "heat of activation" (abbreviated herein as Δ-H_{ACT}) indicates how much energy must be put into a material or mixture in order to get a reaction started along a certain pathway. In a simple analogy, the "heat of activation" is comparable to saying that before a piece of paper or wood will begin to burn, the burning reaction must be started by first putting energy into the system. This can be done by lighting a piece of paper or wood with a match, or some other source of flame or heat.

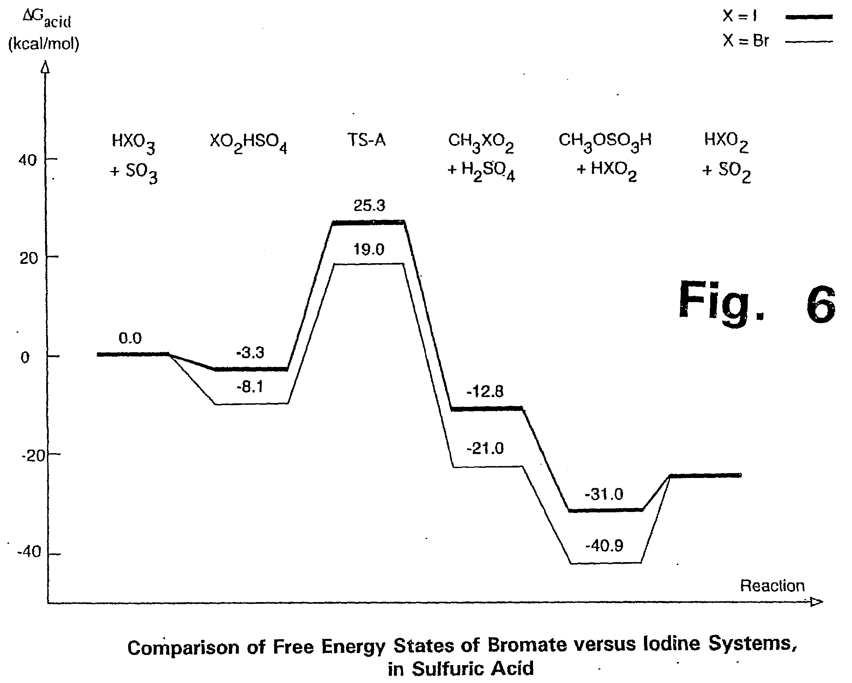

The heat of activation (Δ-H_{ACT}) for a chemical reaction can be shown on a graph, by plotting various calculated numbers in an energy profile, such as shown in FIG. 6. When drawn on this type of graph, the energy state of a starting material (or combination of reagents) begins at a baseline value or starting point that is arbitrarily set at zero. This initial value of zero simplifies the calculations and makes it easier to quickly interpret any numbers that follow.

Starting at a zero point for unreacted reagents, the energy of the starting material or mixture must first climb over some sort of hump, or peak, which requires activation energy to be introduced into the system, to get the reaction started. This initial hump, or peak, represents an increase in energy, and the height of this hump or peak depicts the heat of activation (Δ-H_{ACT}). To use the analogy from above, this initial increase in enthalpy is what happens when a burning match is held to a piece of paper or wood; the flame from the match provides the activation energy that will cause the paper or wood to start burning.

When a material or mixture that is being heated reaches a "transition state" (abbreviated as TS in the graphs), it will begin burning (or otherwise chemically reacting) on its own, in a manner that will release more heat than was required to start the burning process or other reaction. This is what enables a piece of paper or wood to keep burning, and begin releasing heat, once it has started to bum. This reaction is depicted by the large downward slope on the right side of the energy profile that can be drawn by calculating the energy states at each major point along a reaction pathway.

If the reaction is allowed to proceed all the way to completion, the amount of heat that was released by the overall reaction can be measured (or calculated, by computer software). This amount of "total heat release" is usually expressed as the "heat of reaction", abbreviated herein as Δ-H_{RXN}. By convention, it is measured and expressed, not compared to the peak that represents the highest-energy transition state with the heat of activation, but compared to the baseline zero value that applied to the initial, unheated and unreacted material or mixture. Therefore, it represents a 11net" energy output.

The overall (net) heat of reaction (Δ-H_{RXN}) for a chemical reaction, is crucially important in analyzing the reaction, because it indicates how much more "stable" the reaction products are, compared to the unreacted starting material. It provides a numerical indication of how readily and easily a certain material or mixture can be converted to a certain set of desired reaction products. In addition, in a sophisticated computer model that also is able to recognize competing pathways (as well as competing intermediates and products that may be more stable than the desired intermediates and products), this type of modeling also can provide useful indicators of how efficient the yield(s) will be, and the quantities of unwanted byproducts or unreacted reagents that are likely to be generated by the process.

Both of those two values (the heat of activation, Δ-H_{ACT}, and the heat of reaction, Δ-H_{RXN}) are crucial in any complex chemical reaction, and both values must be calculated by a computer program used to model complex chemical reactions.

However, as is known to chemists, Δ-H values tell only half of the story, when a chemical reaction is being modeled on a computer. The other crucial calculation involves Δ-G, a term called "Gibbs free energy", which introduces changes in *entropy* caused by a certain chemical reaction, according to the formula Δ-G = Δ-H-TΔ-S, where T is temperature and Δ-S is the change in entropy. In layman's terms, entropy is a value that indicates unusable or wasted energy, which gets lost to the surrounding environment when a certain chemical reaction occurs. This value is generally related to the change in *entropy* created by a certain chemical reaction. In layman's terms, entropy is a value that indicates unusable or wasted energy, which gets lost to the surrounding environment when a certain chemical reaction occurs, in terms that can be thought of as waste heat, "randomness", or "free energy" (free, not in a good sense, but in the way that would be used by a livestock owner whose cattle or horses, which had been carefully gathered and penned in a corral, have broken down a fence and are now running free). This type of "free" or unusable energy is often referred to by chemists as "overhead", because it is analogous in some respects to taxes, pension costs, time that must be diverted from other work to fill out government forms, and inventory shrinkage due to thefts. These are some of the "overhead" costs of running a business, and if they keep creeping higher and higher until they accumulate to unacceptable or intolerable levels, they can render a business unprofitable and eventually drive it into bankruptcy.

Since calculated Δ-G values represent "overhead" or "unproductive costs," small or negative Δ-G values for a certain reaction indicate that the reaction is promising, and is likely to proceed readily and with good yields. By contrast, high Δ-G values are bad, and indicate that a reaction will be troubled by sluggishness, low yields, AND other problems.

As an example of how Δ-G Values can indicate whether a reaction pathway will proceed efficiently, graphs of several successive Δ-G values, for certain intermediate states that will occur in a bromate-sulfate system as developed by the Applicant, and in an iodine system disclosed in the prior art, is provided in FIG. 6 of this application. The elevated "humps" that represent the transition states (TS) for both pathways indicate that energy must be put into the system to get the reaction started, as discussed above. The negative final values indicate that the reactions can proceed, if they can get past the transition-state "humps"; By comparing the. free energy states of the bromate-sulfate system against the free energy states of the iodine system, as shown in FIG. 6, it becomes clear that the calculations indicate that the bromate-sulfate system offers a more promising system that can probably generate better yields than the iodine system, since the transition state of the bromate-sulfate system is not as high (and therefore not as difficult to reach and cross, and not as likely to drive any reagents or intermediates toward alternate pathways that can be taken with less energy) as the transition state that occurs in the iodine system.

The calculated Δ-H_{ACT}, Δ-H_{RXN}, and Δ-G values for the reaction pathways disclosed herein turn out to be exceptionally or even extraordinarily promising, based on computer modeling done to date. Those calculated numbers are not enclosed with this patent application, since the specific and detailed numbers are not necessary for actually carrying out this invention once the conclusions, pathways, and mechanisms have disclosed, and because the Applicant does not yet have a strong and reliable sense of how heavily and strongly those calculated numbers (which were generated by a graduate student) can be relied upon. Nevertheless, those numbers have been submitted to the U.S. Patent and Trademark Office, in an appendix that was included along with provisional application 60/480,183. The file containing that provisional application, including the appendix with the Δ-H and Δ-G energy calculations, is open for inspection and copying by the public, and anyone is free to inspect those numbers. However, it must be understood that: (i) these computer simulations are not perfect, and must rely on various simplifying and other assumptions, which must be selected as part of the programming before a model of a complex system can be run; (ii) the only real proof of a mode led pathway must come from actual performance of the reaction system, both at small-scale benchtop levels, and in larger scaled-up facilities; and, (iii) this invention does not rest or rely upon computer-modeled Δ-H or Δ-G numbers, and instead rests on the disclosure of new and valuable chemical pathways that can be understood, followed, and used by those skilled in the art, based on the disclosures in this application supplemented by the level of skill in the art among those who specialize in hydrocarbon chemistry.

### POTENTIAL COMPETING REACTIONS

Several potentially competing reactions have been identified by the Applicant that might interfere with the pathway from methane to MSA or methanol, if they were to occur in large quantities. However, computer modeling to date indicates that these potentially competing reactions generally should not create major problems that would seriously hinder the reaction schemes disclosed herein, for the reasons described below. Nevertheless, anyone who tests any of the reaction schemes disclosed herein, either on a small-scale benchtop level or in scale-up testing, should be aware of these potentially competing pathways, so that appropriate steps can be taken to monitor them, and to avoid or minimize them, if necessary, by appropriate means (such as by adding one or more additives or inhibitors, by adjusting one or more reaction parameters to shift a thermodynamic drive or balance away from the unwanted compound(s), by passing a fluidized stream through an immobilized bed that will trap unwanted compounds, etc.).

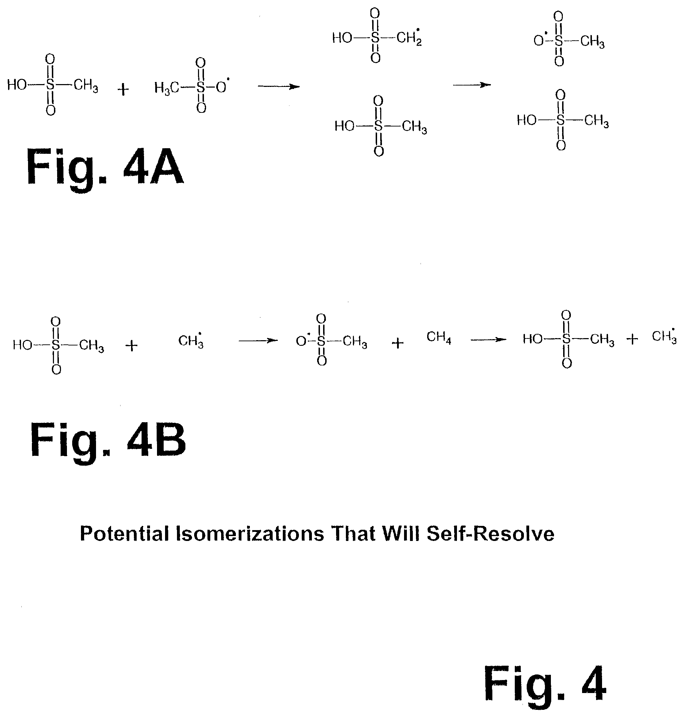

Two potentially competing pathways are shown in FIG. 4, which comprises FIG. 4A and FIG. 4B. In the top pathway, shown in FIG. 4A, a methylsulfonic acid radical (H₃CSO₃*) may react with a completed molecule of MSA, H₃C-SO₃H, in a manner that would effectively (as the net result of a two-step process) transfer one of the hydrogen atoms from a methyl group, on completed methylsulfonic acid, to an oxygen atom on a methylsulfonic radical. This would effectively transfer the highly reactive unpaired electron from an oxygen atom, to a methyl group, as shown in FIG. 4A. However, computer simulations done to date indicate that if this happens, the resulting HO-SO₂-CH₂* radical will either: (i) spontaneously rearrange by itself, or (ii) react with another molecule of MSA, in a way that will transfer the unpaired electron back to the oxygen atom, rather than leaving it on a methyl group. Either rearrangement will regenerate the same type of MSA radical that started the process, where the unpaired electron is on an oxygen atom, rather than on a methyl group.

In another potentially competing pathway, shown in FIG. 4B, a molecule of completed MSA, H₃CSO₃H, might react instead with a methyl radical (*CH₃), in a way that regenerates an MSA radical (CH₃SO₃*) while also regenerating methane (CH₄) from the methyl radical. However, the computer simulations done to date indicate that if this unwanted reaction occurs, the newly-created MSA radical will quickly attack a complete molecule of methane (since fresh quantities of methane presumably are being supplied in bulk to the system at all times), and the MSA radical will remove a hydrogen atom from the methane, to create completed MSA along with a new methyl radical (*CH₃). In a properly-optimized and properly-run system, which constantly supplies more SO₃ to the system while also removing MSA condensate, the new methyl radical will be much more likely to attack an SO₃ molecule that has just entered the system, than a recently completed MSA molecule that is on its way out of the system.

### SYMPHORIC AND ANCHIMERIC SYSTEMS; THE BROMATE-SULFATE SYSTEM

Although not relating to an embodiment of the invention, in order to illustrate a wider context FIGURE 5 depicts a symphoric and anchimeric reaction system developed by the Applicant, using a bi-functional reagent that has both an electrophilic domain (i.e., positively charged, electron-seeking), and a nucleophilic domain (i.e., negatively charged, proton-seeking). To qualify as symphoric and/or anchimeric, those two domains must be (i) contained in the same molecule, and (ii) spaced apart from each other by a distance that will allow the reagent to provoke coordinated proton and electron shifts, in a particular targeted hydrocarbon molecule, in a manner that will create transitional intermediates having reduced thermodynamic barriers. These types of coordinated proton and electron shifts can improve the selectivity and yield of a reaction system for converting an otherwise stable and/or symmetric hydrocarbon (such as methane, or other lower alkanes) into other intermediates or products that are more reactive and easier to handle and work with.

A particular bromate-sulfate reagent, having the formula HO₃S-O-BrO₂, was settled upon by the Applicant as being exceptionally well-suited for exerting symphoric and anchimeric effects on methane. It can drive methane through a reaction pathway that can generate methyl bisulfate (H₃COSO₃H), as shown in FIG. 5. The methyl bisulfate can be hydrolyzed to release methanol, which can be used or sold, and this hydrolysis will also release sulfuric acid, which in this system can be recycled, without forming waste, to regenerate the bromate-sulfate reagent.

Accordingly, the bromate-sulfate reagent and system shown in FIG. 5 are disclosed as an exemplary system for converting methane into other products, such as methanol. Although the radical-initiator system (using Marshall's acid) that was subsequently developed by the Applicant is believed to offer a better pathway for converting methane into methanol, the bromate-sulfate system is believed to offer a better pathway than any other system that was known, prior to this discovery by the Applicant. Accordingly, as mentioned above, it should be studied carefully by any organic chemists who are interested in methane or other lower hydrocarbons, since it may offer a powerful set of tools and options that may be able to achieve various useful results, in a manner that will be complementary to the radical initiator system, and that may be applicable to various situations where a radical initiator system will not work (such as, in particular, when dealing with mixtures of reagents, in which highly reactive free radicals would react with too many different compounds, thereby providing low and unsatisfactory selectivities and yields).

The bromate-sulfate reagent (or any other similar symphoric and/or anchimeric reagent having both electrophilic and nucleophilic domains, spaced apart from each other a controlled distance) can also be computer-modeled and laboratory-tested, to evaluate its ability to manipulate ethane, propane, or any other lower alkane molecule (as used herein, terms such as "lower alkane" or "lower hydrocarbon" include compounds that have up to four carbon atoms). Such symphoric and/or anchimeric reagents can also be modeled and/or tested with any other type of compound that contains carbon and hydrogen atoms (the term "hydrocarbon is used broadly herein, to include any compound that contains both hydrogen and carbon atoms, regardless of whether such compound also contains oxygen, sulfur, nitrogen, or any other element). Candidate compounds include (i) substituted alkanes; (ii) cycloalkanes, include hetero and/or substituted cycloalkanes that have non-carbon, non-hydrogen atoms, either as part of the ring, or attached to the ring; (iii) aromatic hydrocarbons; and (iv) unsaturated hydrocarbons.

### EXAMPLE 1: EQUIPMENT AND REAGENTS

All tests described below were done in the laboratories of Prof. Ayusman Sen, in the Chemistry Department at Pennsylvania State University. All experiments were carried out under inert gas (nitrogen, N₂) in either a glove box or a glovebag.

Except as noted below, the reactions were carried out in a sealed vessel designed to withstand high pressures (these devices are commonly referred to in chemistry labs as "bombs"), containing a glass liner (this liner, which can be easily removed for thorough cleaning and sterilization, will not break when high pressures are reached inside the bomb, because the pressures will be equal on both sides of the glass walls of the liner). The bomb used has a 0.95 cm (3/8 inch) stainless steel walls, and an internal chamber 3.81 cm (1.5 inches) in diameter and 11.43 cm (4.5 inches) high. The glass liner had an internal diameter of 3.15 cm (1.24 inches), a height of 10.16 cm (4 inches) and a wall thickness of 0.16 cm (1/16 inch). A stirring bar was used in some experiments, 2.54 cm (1 inch) long and having a roughly circular cross section of 0.48 cm (3/16 inch) diameter.

In a number of experiments, a vial was placed inside the liner, to ensure that there was no direct mixing of a first liquid that was loaded into the bottom of the glass liner, and a second liquid that was loaded into the vial. The vial that was used had a 2.54 cm (1 inch) outside diameter, a wall thickness of 0.1.6 cm (1/16 inch), and a height of 5.72 cm (2.25 inches). The diameter of the opening on top (with external threads, to accommodate a screw cap) was 1.59 cm (5/8 inch). A small stirring bar was sometimes placed inside the vial, with a length of 1.27 cm (1/2 inch) and a diameter of 0.32 cm (1/8 inch).

### EXAMPLE 2: PREPARATION OF MARSHALL'S ACID

To prepare Marshall's acid (peroxy-disulfuric acid), a gaseous mixture of SO₃, in inert nitrogen (N₂) was loaded into a vessel containing a solution of 70% hydrogen peroxide in water, at 13 to 15°C. The reaction was continued, with stirring, until essentially all liquid reagents had been consumed, which was confirmed by the presence of a consistent viscous solution with some solid crystals present, but with no inhomogeneous liquids.

In Run #1, 6.9 g (86.3 mmol) of S0₃ was absorbed in 1.1 g of 70% H₂O₂ (22.7 mmol) in water (17.7 mmol), for 5.5 hours. After accounting for the diversion of some SO₃ into H₂SO₄, the molar ratio of SO₃ to H₂O₂ was 3:1. It was presumed that all H₂O₂ was converted to Marshall's acid (H₂S₂O₈), and all water was converted to H₂SO₄. These calculations and assumptions indicated that the solution contained Marshall's acid at 22.7 mmol (56.2% of the total solution, by weight), and sulfuric acid at 17.7 mmol (21.3%), with unreacted SO₃ present at 23.2 mmol (22.5%).

In Run #2, 5.2 g (65 mmol) of SO₃ was absorbed in 1.2 g of 70% H₂O₂ (25 mmol) in water (19.4 mmol), for 5.5 hours. Since the molar ratio of SO₃ (after subtracting a number of mmol of SO₃ for formation of H₂SO₄) to H₂O₂ is 1.8:1, it was presumed that the first equivalent of SO₃ reacted with H₂O₂ to form Caro's acid (H₂SO₅), and the rest of the 0.8 equivalent of SO₃ reacted with the Caro's acid to form Marshall's acid. These assumptions indicated a solution of Marshall's acid at 20.6 mmol (62.5%), sulfuric acid at 19.4 mmol (29.7%), and Caro's acid at 4.4 mmol (7.8%).

In Run #3, 8.3 g (103.8 mmol) of SO₃ was absorbed in 1.8 g of 70% H₂O₂(37.0 mmol) in water (30.0 mmol), for 7 hours. After subtracting a number of mmol of SO₃ for formation of H₂SO₄, the molar ratio of SO₃ to H₂O₂ was 2:1. This indicated a solution of Marshall's acid at 37.0 mmol (71.3%), and sulfuric acid at 30 mmol (28.7%).

In Run #4, 8.3 g (103.8 mmol) of SO₃ was absorbed in 2.1 g of 70% H₂O₂ (43.2 mmol) in water (35 .0 mmol), for 7 hours. The molar ratio of SO₃ (after accounting for H₂SO₄ formation) to H₂O₂ was 1.6:1. This indicated a solution of Marshall's acid at 25.6 mmol (47.7%), sulfuric acid at 35 mmol (33%), and Caro's acid at 17.6 mmol (19.2%).

### EXAMPLE 3: PROCEDURES FOR TESTING MSA FORMATION

The tests described below uses MSA/ SO₃ mixtures as the liquid media. Gaseous SO₃ can be efficiently absorbed in methanesulfonic acid (MSA), at ratios up to about 10: 1, so a solution of SO₃ absorbed in liquid MSA was placed in a glass vial, as described above. 1 to 2 grams of a Marshall's acid solution (prepared as described in Example 2) was placed in the same vial.

The vial was placed inside the somewhat larger glass liner (also described above) in the bomb, and 3 to 5 g of stabilized liquid SO₃ was loaded into the liner.

This approach (dividing the SO₃ into two separate zones) was taken to prevent the Marshall's acid from being overloaded with SO₃, since high concentrations of SO₃ can trigger degradation of Marshall's acid, causing it to release oxygen, and destroying its peroxide bond.

The bomb was sealed and pressurized with 55-97 bar (800-1400 psi) of methane. It was heated to 48-52°C, and the pressure was monitored as it dropped. Heating was continued until the pressure no longer continued to fall, and reached an asymptotic level.

The bomb was then allowed to cool gradually to room temperature, over a couple of hours. The pressure was released slowly, the bomb was opened, and the solution in the vial was diluted with 5-10 mL of water. The liquid was then analyzed, via ¹H nuclear magnetic resonance (NMR).

In most cases, MSA was the only product that was found in the liquid phase. It was quantified, using integration of peak intensity compared to a standard peak of dimethyl sulfoxide, in a capillary tube, to confirm that additional MSA had indeed been formed, in addition to the MSA that was already present in the liquid that was initially loaded into the vial.

The gas mixture that was present in the cooled bomb was also analyzed, by gas chromatography. No carbon dioxide was detected in the gas phase, in any of the test runs.

### EXAMPLE 4: FIRST RUN: METHANE YIELD 40.4%, SO₃ YIELD 96.0%

In the first reaction test that was run as described above, 1.0 gram of the Marshall's acid preparation described in Run #1 (above) was used (with Marshall's acid at 56.2%, sulfuric acid at 21.3%, and SO₃ at 22.5%). This was added to a vial containing 50 mmol of MSA and 63 mmol of SO₃. 2.8 g (35 mmol) of stabilized liquid SO₃ was added to the liner, outside the vial. The bomb was pressurized to 55 bar (800 psi) with purified methane, and heated at 48-52°C.

4.8 bar (70 psi) of pressure drop was observed within 2 hours, and the vessel was recharged with 3.45 additional bar (50 additional psi) of methane. The total pressure drop rose 8.3 bar (120 psi) the next 2 hours (i.e., after 4 hours total), and the vessel was recharged with an additional 3.45 bar (50 psi) of methane. The total pressure drop was 17 bar (250 psi) over 14 hours.

The total methane that was injected into the bomb was measured and calculated at 240 mmol, and the total amount of SO₃ in the liquid media (i.e., dissolved in MSA and placed inside the vial) was 101 mmol.

The yield of newly-formed MSA was measured and calculated to be 97 mmol (147 mmol total, minus 50 mmol already present in the MSA/SO₃ liquid media).

This indicated a methane conversion yield of 40.4%, and an SO₃ conversion yield of 96.0%.

### EXAMPLE 5: SECOND RUN: METHANE YIELD 40.6%, SO₃ YIELD 99.1%

In the second reaction test that was run as described above, 1.0 g of Marshall's acid solution from Run #2 (above) was loaded into a vial containing 48 mmol of MSA and 71 mmol of SO₃. 3.0 g (38 mmol) of stabilized liquid SO₃ was loaded into the liner, outside the vial. The bomb was pressurized with 69 bar (1000 psi) of methane, and heated at 48-52°C.

6.9 bar (100 psi) of pressure drop was observed after 2 hours; 10.3 bar (150 psi) of pressure drop was observed after 4 hours; and 19 bar (280 psi) of pressure drop was observed after 12 hours.

The total methane in the bomb was measured and calculated to be 266 mmol. The total SO₃ in the liquid media was calculated at 109 mmol. The yield of MSA was measured and calculated to be 108 mmol (156 mmol minus 48 mmol).

This indicated a methane conversion yield of 40.6%, and an SO₃ conversion yield of 99.1 %.

### EXAMPLE 6: THIRD RUN: METHANE YIELD 43.3%, SO₃ YIELD 92.6%

In the third reaction test that was run as described above, 1.5 g of Marshall's acid solution from Run #3 (above) was loaded into a vial containing 43 mmol of MSA and 99 mmol of SO₃. 4.0 g (50 mmol) of stabilized liquid SO₃ was loaded into the liner, outside the vial. The bomb was pressurized with 83 bar (1200 psi) of methane, and heated at 48-52°C.

10.3 bar (150 psi) of pressure drop was observed after 2 hours; 17 bar (250 psi) of pressure drop was observed after 4 hours; and 21 bar (300 psi) of pressure drop was observed after 10 hours.

The total methane in the bomb was measured and calculated to be 319 mmol. The total SO₃ in the liquid media was calculated at 149 mmol. The yield of MSA was measured and calculated to be 138 mmol (181 mmol minus 43 mmol).

This indicated a methane conversion yield of 43.3%, and an SO₃ conversion yield of 92.6%.

### EXAMPLE 7: FOURTH RUN: METHANE YIELD 33.6%, SO₃ YIELD 92.6%

In the fourth reaction test that was run as described above, 2.4 g of Marshall's acid solution from Run #4 (above) was loaded into a vial containing 43 rnmol of MSA and 77 mmol of SO₃. 4.6 g (58 mmol) of stabilized liquid SO₃ was loaded into the liner, outside the vial. The bomb was pressurized with 97 bar (1400 psi) of methane, and heated at 48-52°C.

6.9 bar (100 psi) of pressure drop was observed after 1 hours; 12.4 bar (180 psi) of pressure drop was observed after 2 hours; 16.5 bar (240 psi) of pressure drop was observed after 3 hours; and 21 bar (300 psi) of pressure drop was observed after 6 hours.

The total methane in the bomb was measured and calculated to be 372 mmol. The total SO₃ in the liquid media was calculated at 135 mmol. The yield of MSA was measured and calculated to be 125 mmol (168 mmol minus 43 mmol).

This indicated a methane conversion yield of 33.6%, and an SO₃ conversion yield of 92.6%.

Upon examination and comparison of these data, it appeared that the concentration of methane in the bomb was the rate determining factor, since increasing methane pressure increased the rate of the reaction. According to our previous calculation, the molar concentration of CH4 (83 bar) in H20 at 50°C is 0.078M, solubility of CH4 could be higher in MSA/S03 mixture, but it is still quite low comparison with the amount of S03 in the liquid phase. Therefore the key step for increasing rate of the reaction is increasing the solubility of CH4 in liquid phase.

In addition, various calculations (including a calculated rate constant of 3.0 x 10⁻⁵ per second, for homolysis of Marshall's acid in SO₃ at 50°C) indicated that the rate of conversion of methane to MSA is around 20 times faster than the rate of homolysis of Marshall's acid. This helps explain why the pressure continued to drop over spans of 10 hours (in the laboratory test conditions that were used) and why conversion of SO₃ was in very high ranges, up to 99%. When scaled up to industrial levels, where continuous-flow devices that are designed for high throughput levels (such as loop reactors and/or spinning bed reactors) are used instead of small volume batch reactors, it is anticipated that efficient and economical reaction levels can be achieved in minutes, or even seconds, rather than over a span of hours.

### EXAMPLE 8: NO CONVERSION BY POTASSIUM SALT OF MARSHALL'S ACID

As a comparative experiment, 270 mg of the potassium salt of Marshall's acid (K₂S₂O₈; 1.0 mmol) was loaded into the vial, and 13.5 g of stabilized SO₃ was loaded into the liner, using procedures identical to the testing of the free acid form of Marshall's acid, as disclosed above. The bomb was pressurized with 55 bar (800 psi) of methane, and heated at 48-52°C for 20 hours. However, no pressure drop was observed. Then temperature was then increased to 75-80°C, for an additional 16 hours, but still no pressure drop was observed.

The absence of any pressure drop indicates that the potassium salt of Marshall's acid failed to initiate any reaction between the methane, and the SO₃.

Thus, there has been shown and described a new and useful means for creating methanol from methane, and for creating other useful and valuable lower alkane derivatives, intermediates, and products, from methane and other lower alkane molecules. Although this invention has been exemplified for purposes of illustration and description by reference to certain specific embodiments, it will be apparent to those skilled in the art that various modifications, alterations, and equivalents of the illustrated examples are possible. Any such changes which derive directly from the teachings herein, and which do not depart from the spirit and scope of the invention, are deemed to be covered by this invention.

### REFERENCES

Basickes, N., et al, "Radical-initiated functionalization of methane and ethane in fuming sulfuric acid," J Am Chem Soc 118: 13111-13112 (1996)
Broekhuis, R.R., et al, "The ejector-driven monolith loop reactor- experiments and modeling," Catalysts Today 69: 887-893 (2001)
Gilbert, T.M., et al, "Comparison between oxidative addition and o-bond methasis as possible mechanisms for the Catalytica methane activation process by platinum(II) complexes: A density functional theory study," Organometallics 20: 1183-1189 (2001)
Gilbert, G.E., Sulfonation and Related Reactions (Interscience Publishers, 1965)
Golombok, M., et al, "A chemical alternative to natural gas flaring," Ind Eng Chem Res 42: 5003-5006 (2003)
Lin, M., et al, "Oxidation and oxidative carbonylation of methane and ethane by hexaono-u-peroxodisulfate(2-) ion in aqueous medium: A model for alkane oxidation through the hydrogen-atom abstraction pathway,"J Chem Soc Chem Comm 1992: 892-893 (1992)
Lin, M. & Sen, A., Nature 368: 613 (1994)
Lin, M. et al, J. Am. Chem. Soc. 118: 4574 (1996)
Lobree, L.J., et al, "K2S2O2-initiated sulfonation of methane to methanesulfonic acid," Ind Eng Chem Res 40: 736-742 (2001)
Mukhopadhyay, S., et al, "Effects of solvent acidity on the free-radical-initiated synthesis of methanesulfonic acid from CH4 and SO3," American Chemical Society 2002: A-E (2002)
Mukhopadhyay, S., et al, "A novel method for the direct sulfonation of CH4 with SO3 in the presence of KO2 and a promoter," Organic Process Research & Development 2003: A-D (2003)
Mukhopadhyay, S., et al, "A high-yield approach to the sulfonation of methane to methanesulfonic acid initiated by H2O2 and a metal chloride," Angew Chem Int Ed 42: 2990-2993 (2003)
Mukhopadhyay, S., et al, "Effects of solvent acidity on the free-radical-initiated synthesis of methanesulfonic acid from CH4 and SO3," Amer Chem Soc 2002: A-E (2002)
Nizona, G.V., et al, "Carboxylation of methane with CO or CO2 in aqueous solution catalysed by vanadium complexes," Chem Commun 1998: 1885-1886 (1998)
Periana, R.A., et al, "A mercury-catalyzed, high-yield system for the oxidation of methane to methanol," Science 259: 340 (1993)
Periana, R.A., et al, "High yield conversion of methane to methyl bisulfate catalyzed by iodine cations," Chem Commun 2002: 2376-2377 (2002)
Periana, R.A., et al, "Platinum catalysts for the high-yield oxidation of methane to a methanol derivative," Science Magazine 280: 560-564 (1998)
Reis, P.M., et al, "Single-pot conversion of methane into acetic acid in the absence of CO and with vanadium catalysts such as amavadine," Angew Chem Int Ed 42: 821-823 (2003)
Zerella, M., et al, "Synthesis of mixed acid anhydrides from methane and carbon dioxide in acid solvents," Amer Chem Society 5: 3193-3196 (2003)
Ziegler, T., "Approximate density functional theory as a practical tool in molecular energetics and dynamics," Chem Reviews 91: 651-67 (1991)

## Claims

1. A method for converting methane into methanesulfonic acid, comprising the following steps:
a. removing hydrogen atoms from methane, thereby generating methyl radicals, each having an unpaired electron;
b. contacting the methyl radicals with sulfur trioxide, under conditions that enable the methyl radicals to react with the sulfur trioxide in a manner that forms methylated oxide radicals having sufficient reactivity to remove hydrogen atoms from methane; and,
c. reacting the methylated oxide radicals with methane, under conditions that enable the methylated oxide radicals to remove hydrogen atoms from the methane, thereby forming methanesulfonic acid while also generating newly-formed methyl radicals,
wherein said steps are initiated by a radical initiator compound and then sustained on a steady-state basis by adding appropriate quantities of methane and the sulfur trioxide to a reactor device.

2. The method of claim 1, wherein said steps are carried out using anhydrous reagents.

3. The method according to any of the preceding claims, wherein said radical initiator compound is a peroxo-acid.

4. The method according to claim 3, wherein said peroxo-acid is generated using hydrogen-peroxide.

## Patentansprüche

1. Verfahren zum Überführen von Methan zu Methansulfonsäure, umfassend die folgenden Schritte:
a. Entfernen von Wasserstoffatomen von Methan, wodurch Methylradikale erzeugt werden, von denen jedes ein ungepaartes Elektron aufweist;
b. Inkontaktbringen der Methylradikale mit Schwefeltrioxid unter Bedingungen, die es den Methylradikalen ermöglichen, mit dem Schwefeltrioxid auf eine Weise zu reagieren, dass methylierte Oxidradikale gebildet werden, die ausreichende Reaktivität aufweisen, um Wasserstoffatome von Methan zu entfernen; und
c. Umsetzen der methylierten Oxidradikale mit Methan unter Bedingungen, die es den Methylradikalen ermöglichen, Wasserstoffatome von dem Methan zu entfernen, wodurch Methansulfonsäure gebildet wird, während außerdem neu gebildete Methylradikale erzeugt werden,
wobei die Schritte von einer Radikalinitiatorverbindung initiiert und dann durch Zugabe geeigneter Mengen von Methan und des Schwefeltrioxids zu einer Reaktorvorrichtung auf Steady-State-Basis beibehalten werden.

2. Verfahren nach Anspruch 1, wobei die Schritte unter Verwendung von wasserfreien Reagenzien ausgeführt werden.

3. Verfahren nach einem der vorhergehenden Ansprüche, wobei es sich bei der Radikalinitiatorverbindung um eine Peroxosäure handelt.

4. Verfahren nach Anspruch 3, wobei die Peroxosäure unter Verwendung von Wasserstoffperoxid erzeugt wird.

## Revendications

1. - Procédé pour convertir du méthane en acide méthanesulfonique, comprenant les étapes suivantes :
a. retrait d'atomes d'hydrogène à partir du méthane, permettant ainsi de générer des radicaux méthyle, chacun ayant un électron non apparié ;
b. mise en contact des radicaux méthyle avec du trioxyde de soufre, dans des conditions qui permettent aux radicaux méthyle de réagir avec le trioxyde de soufre d'une manière qui forme des radicaux oxyde méthylé ayant une réactivité suffisante pour retirer des atomes d'hydrogène à partir du méthane ; et
c. réaction des radicaux oxyde méthylé avec du méthane, dans des conditions qui permettent aux radicaux oxyde méthylé de retirer des atomes d'hydrogène à partir du méthane, permettant ainsi de former de l'acide méthanesulfonique tout en générant également des radicaux méthyle nouvellement formés,
lesdites étapes étant initialisées par un composé initiateur de radicaux, puis entretenues sur une base d'état stable par addition de quantités appropriées de méthane et du trioxyde de soufre dans un dispositif réacteur.

2. - Procédé selon la revendication 1, dans lequel lesdites étapes sont effectuées à l'aide de réactifs anhydres.

3. - Procédé selon l'une quelconque des revendications précédentes, dans lequel ledit composé initiateur de radicaux est un peroxo-acide.

4. - Procédé selon la revendication 3, dans lequel ledit peroxo-acide est généré à l'aide de peroxyde d'hydrogène.
